# EUROPEAN PATENT SPECIFICATION

(11) **EP 4 204 060 B1**
(45) Date of publication and mention of the grant of the patent: **12.11.2025**
(21) Application number: 21790623.9
(22) Date of filing: 30.08.2021
(51) Int. Cl.: A61M 25/02

(54) **MEDICAL DEVICE STABILIZATION SYSTEM**
STABILISIERUNGSSYSTEM FÜR EINE MEDIZINISCHE VORRICHTUNG
SYSTÈME DE STABILISATION DE DISPOSITIF MÉDICAL

(30) Priority: 31.08.2020 US 202063072776 P
(43) Date of publication of application: 05.07.2023
(73) Proprietor: AVACEN, Inc., Carlsbad, CA 92009 (US)
(72) Inventor: BONAGUIDI, Michael, O., Jr., California 91913 (US); KLICH, Melanie, California 90803 (US); BRODT, Thomas, California 90803 (US); QUINTANILLA, Jorge, California 93003 (US); ALZATE, Gregg D., California 92014 (US)
(74) Representative: Vossius & Partner Patentanwälte Rechtsanwälte mbB
(86) International application number: PCT/US2021/048279
(87) International publication number: WO 2022/047329

(56) References cited:
- EP-B1- 2 804 655
- WO-A1-2014/149668
- JP-A- 2008 220 633
- US-A- 5 484 420
- US-A1- 2009 149 814
- US-A1- 2014 364 810
- US-B2- 9 895 514

## Description

### CROSS-REFERENCE TO RELATED APPLICATIONS

### FIELD OF THE INVENTION

This application relates generally to devices and systems for securing medical instruments or devices to a patient.

### BACKGROUND

Medical devices, such as catheters, are widely used to access the internal body lumen of a patient. Such devices are commonly used to create direct vascular access for medical device placement, hemodynamic monitoring, pharmaceutical injections, infusion therapies, and a wide and rapidly growing range of interventional procedures. These procedures often require repeated and/or sustained access to the body lumen and may be partially or wholly implanted, e.g., subcutaneously, percutaneously, or transcutaneously, at sites throughout the body e.g., femoral, popliteal, brachial, axial, tibial, and carotid. After access is established, it is critical that the device is secured to prevent unintentional motion or displacement. Often, the extracorporeal portion of the catheter, adjacent to the insertion site, is secured to the patient via adhesive tape, sutures, or catheter-specific securement devices. As the catheter enters the patient at an angle, the sudden bend of the catheter external to the patient in order to secure the catheter parallel to the patient's body may cause insertion site stress and discomfort to the patient, and also excessive bleeding.

It is common for patients with transcutaneously situated catheters to experience bleeding at the insertion site of the catheter. Without proper support and stabilizing systems readily available to stop the bleeding, medical professionals may resort to wedging objects between the catheter and the patient's skin adjacent to the catheter insertion site so that the catheter will enter the patient's body at the insertion angle, to reduce or stop the bleeding. This is most often done in response to insertion site bleeding, necessitating hemostatic intervention and in some cases repositioning and re-procedure which could be avoided with a proper catheter stabilizing system.

Moreover, the repeated manipulation and movement of the extracorporeal portion of the catheter causes wear and damage to the catheter, as well as communicates motion to the patient's skin adjacent to the catheter insertion site, thereby causing various complications depending upon the type of catheter used. To stabilize the extracorporeal portion of the catheter, tie-down materials, such as bandaging, patches with upstanding anchoring posts, medical adhesive tape, belts, elastic bands, and sutures, are typically used, all of which do not rid of the complications described above with regard to patient discomfort and bleeding.

Devices have been theorized for securing and/or stabilizing a catheter to a patient. For example, U.S. 5,484,420 to Russo describes a retention bolter for a percutaneous catheter which has a convexly curved exterior surface which contacts the epidermal surface of the patient. The retention bolster slides over the catheter until it contacts the patient's skin at the catheter exit site. When secured in place, the retention catheter rocks along contacting portions between its convexly curved surface and the patient's skin in response to movement of the catheter about the exit site, thereby alleviating pressure that would otherwise be applied by the movement. However, the retention bolster described therein does not accommodate varying insertion angles of various catheters and would potentially cause unwanted bending and/or kinking of the catheter.

U.S. 6,332,874 to Eliasen describes a stabilization sleeve having stabilization wings for receiving a catheter to provide axial and bending strain relief to the catheter. The lumen of the stabilization sleeve may be slightly angled; however, the stabilizer sleeve does not accommodate varying insertion angles of various catheters. U.S. 7,635,354 to Navarro describes a device for fixing a catheter to the body of a patient. The device includes a housing which can be closed by a lid, the housing having a first chamber for passing the portion of the catheter that enters the patient's vein, and a second chamber for accommodating and maintaining the catheter. The device described therein does not accommodate for the insertion angle of the catheter.

U.S. 9,486,613 to Dickert describes a catheter securement device that has a flexible base member and a single piece elastomeric anchoring member mounted to an adhesive side of the base member. The anchoring member has a pair of opposing pull tabs that may be pulled to open a slit of the anchoring member for receiving a catheter hub therein. The anchoring member includes a third tab that is insertable into the slit for securing the catheter hub between the anchoring member and the base member. The catheter securement device described therein does not accommodate for the insertion angle of the catheter.

U.S. 2009/0149814 to Bailey describes a stabilization and support for a catheter that provides stress distribution for loads otherwise imposed on the patient's skin causing damage and sores. The support includes a mount sized to support a catheter extending substantially tangentially to the surface of the patient's skin, thus permitting stabilization of the catheter without exposing the skin to the sharp, highly stressful edges of catheter equipment. The support described therein is a one-sided ramp which may be prone to kinking of the catheter at the top of the support.

U.S. 9,895,514 to Bierman describes a securement system that supports and secures a catheter to the patient's skin. The support system is composed of a base and multiple inclined surfaces extending tangentially upwards to interface with a catheter connector of varying diameter which can be used in conjunction with adhesive to prevent dislodging of the catheter. The support described therein is a one-sided series of ramps which may be prone to kinking of the catheter at the top of the support. The system described therein also does not allow for repositioning of the system in the event that hemostatic intervention is required. JP2008220633 discloses yet another securement device comprising two top surfaces, one presenting a 45° degree slope with relation to the skin's surface and the second being parallel to said surface.

In view of the foregoing, it would be desirable to provide a device for securing an intraluminal medical device to a patient at the angle of insertion to thereby reduce patient discomfort, adverse bleeding events, and protect the instrument or device.

### SUMMARY OF THE INVENTION

In accordance with the principles of the present invention, devices and systems are provided for securing a medical device, e.g., a catheter, to a patient, relative to the angle and point of insertion, thereby reducing stress at the insertion site and discomfort for the patient while minimizing risk of damage to the medical device.

The stabilization system includes a variety of aspects such as a conformable base and/or a stabilizer or support pad. For example, the stabilizer includes a bottom surface, a first top surface extending from a first end of the bottom surface to an apex of the stabilizer, and a second top surface extending from the apex of the stabilizer to a second end of the bottom surface. The stabilizer may be affixed to a skin of the patient such that the first top surface is adjacent to an insertion site of the patient. Accordingly, the medical device may extend from the insertion site along the first top surface over the apex and along the second top surface of the stabilizer toward the patient to thereby secure the medical device to the patient.

The bottom surface of the stabilizer may include an adhesive for adhering the stabilizer directly to the skin of the patient. In addition, at least one of the first top surface or the second top surface may have a concave geometry. The first top surface of the stabilizer may extend from the first end of the bottom surface at an angle corresponding with an insertion angle of the medical device into the patient, to thereby reduce bleeding. Additionally, the first top surface of the stabilizer may extend from the first end of the bottom surface at an angle larger than an angle at which the second top surface extends from the second end of the bottom surface. For example, the first top surface may extend from the first end of the bottom surface at an angle of 20 to 40 degrees, preferably 30 degrees, and the second top surface may extend from the second end of the bottom surface at an angle of 10 to 30 degrees, preferably 20 degrees. The stabilizer may be composed of, e.g., a closed cell foam, thermoplastic, rubber, or other elastomeric or plastic material. In addition, the apex of the stabilizer may have a radius of curvature sized and shaped to prevent kinking of the medical device. The apex of the stabilizer may be at least one inch from the bottom surface of the stabilizer.

The stabilization system further includes one or more straps and/or sites for interfacing with the medical device, e.g., catheter hub, that may be removably coupled to the stabilizer such that the medical device is positioned between the stabilizer and the one or more straps and/or interfaces. For example, one or more straps may be removably coupled to the stabilizer such that the medical device is positioned between the stabilizer and the one or more straps. The one or more straps may be removably coupled to the stabilizer over at least one of the first top surface or the second top surface. Moreover, the one or more straps may include a primary mating surface, and lateral sides of the stabilizer may include a secondary mating surface that mates with the primary mating surface. An end of the one or more straps may be fixed to a first lateral side of the stabilizer, and an opposite end of the one or more straps may be removably coupled to a second lateral side of the stabilizer.

The stabilization system further may include a base having a bottom side and a top side. The bottom side of the base may be affixed to the skin of the patient, e.g., an anterior quadriceps, and the top side may include a first mating surface. The base further may include an opening sized and shaped to receive the medical device therethrough. Accordingly, the bottom surface of the stabilizer may include a second mating surface that may mate with the first mating surface of the base such that the stabilizer is affixed to the skin of the patient via the base and the first top surface of the stabilizer is adjacent to the opening. The bottom side of the base may include an adhesive for adhering the base to the skin of the patient. Additionally, the base may be sized and shaped to be affixed to a vascular access site of the patient selected from a group consisting of a femoral, popliteal, brachial, axial, tibial, or carotid. The base may include one or more fingers, e.g., two fingers, defining the opening. The two fingers may be coupled to one or more sutures via an external sterile adhesive. Moreover, the base may include a plurality of wings extending from a lateral side of the base. For example, a first wing of the plurality of wings may have a length smaller than a length of a second wing of the plurality of wings.

The stabilizer may be decoupled from the base, reoriented 180 degrees, and recoupled to the base. The stabilization system may be used to secure a wide variety of medical devices which require vascular access, for example a ventricular support catheter (e.g. Impella^{®} made available by Abiomed, Danvers, Massachusetts), an intra-aortic balloon pump, an extracorporeal membrane oxygenation (ECMO) machine, a device for catheter-assisted thrombolysis (e.g. Ekos^{®} made available by EKOS Corporation, Bothell, Washington), a temperature control therapy device (e.g. InnerCool^{™} made available by Zoll Medical, Chelmsford, Massachusetts), a cardiopulmonary support catheter, a catheter-directed thrombolysis system, a pulmonary artery catheter (also known as a Swan-Ganz catheter), or a temporary pacing catheter.

In accordance with another aspect of the present disclosure, a method for securing a medical device to the patient is provided. The method may include selecting the stabilization system; and affixing the stabilization system to a skin of the patient such that the medical device extends from an insertion site of the patient along the first top surface, over the apex, and along the second top surface of the stabilizer toward the patient to thereby secure the medical device to the patient. For example, the stabilization system may be affixed to the skin of the patient by affixing the base to the skin of the patient and coupling the stabilizer to the base such that the top surface of the stabilizer is adjacent to the opening of the base.

The invention is defined by claims 1,8. Dependent claims defined preferable embodiments of the invention.

### BRIEF DESCRIPTION OF THE DRAWINGS

FIG. 1 is a perspective view of an exemplary medical device stabilization system constructed in accordance with the principles of the present invention.
FIG. 2 illustrates various views of an exemplary base constructed in accordance with the principles of the present invention.
FIG. 3 illustrates various views of an exemplary stabilizer pad constructed in accordance with the principles of the present invention.
FIG. 4 shows the stabilization system of FIG. 1 in use with a patient having a catheter secured thereon.
FIG. 5 illustrates various views of an alternative exemplary base constructed in accordance with the principles of the present invention.

### DETAILED DESCRIPTION OF THE INVENTION

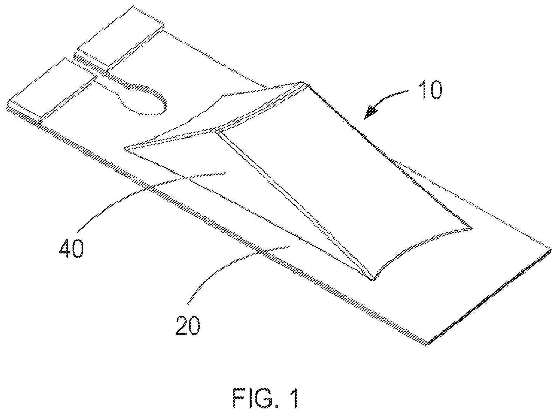

Devices and methods are provided for securing a medical device, e.g., a catheter, to a patient, relative to the angle and point of insertion thereby reducing stress at the insertion site and discomfort for the patient while minimizing risk of damage to the medical device. Referring now to FIG. 1, an exemplary stabilization system 10 is provided. System 10 may be affixed to the patient's skin, e.g., on the patient's anterior quadriceps, to secure and stabilize the extracorporeal portion of a medical device to the patient, such that various medical devices coupled thereto may be easily repositioned. System 10 may be used to secure devices including a ventricular support catheter (e.g. Impella^{®} made available by Abiomed, Danvers, Massachusetts), an intra-aortic balloon pump, an extracorporeal membrane oxygenation (ECMO) machine, a device for catheter-assisted thrombolysis (e.g. Ekos^{®} made available by EKOS Corporation, Bothell, Washington), a temperature control therapy device (e.g. InnerCool^{™} made available by Zoll Medical, Chelmsford, Massachusetts), a cardiopulmonary support catheter, a catheter-directed thrombolysis system, a pulmonary artery catheter (also known as a Swan-Ganz catheter), or a temporary pacing catheter. In addition, system 10 may be used in conjunction with percutaneous suture-mediated closure devices (e.g. Perclose ProGlide^{™} made available by Abbott Laboratories, Chicago, Illinois), as described in further detail below. As shown in FIG. 1, system 10 includes base 20 for adhering to a patient's skin adjacent to the medical device insertion site and stabilizer 40 which may be removably coupled to base 20.

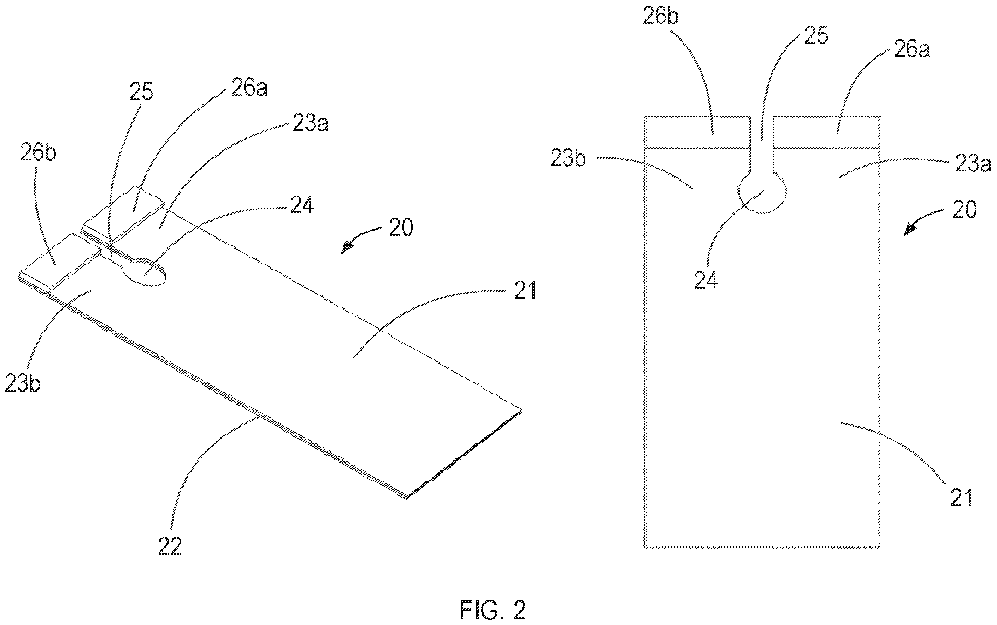

Referring now to FIG. 2, an exemplary base is provided. Base 20 may have a rectangular shape, and may be sized and shaped to be affixed to various parts of the patient's body, and to accommodate particular anatomy and constraints in consideration of the underlying tissues. For example, when sized to be affixed to the patient's anterior quadriceps, base 20 may have a width of three to five inches, and a length of seven to nine inches. As will be understood by a person of ordinary skill in the art, the dimensions and geometry of base 20 may be selected to comfortably adhere to the patient's body in the desired location adjacent to the device insertion site. As shown in FIG. 2, base 20 includes opening 24. Opening 24 may be sized and shaped to receive various sized devices depending on the underlying procedure. Moreover, as shown in FIG. 2, opening 24 may be defined by fingers 23a, 23b. Accordingly, opening 24 may be in communication with slit 25 between fingers 23a, 23b, such that opening 24 may receive the medical device via slit 25 before base 20 is affixed to the patient's body.

Base 20 may include top side 21 and bottom side 22. Bottom side 22 may be affixed to the patient's skin using methods known in the art, e.g., adhesion. For example, bottom side 22 may include a biocompatible and durable adhesive layer. The adhesive layer may be a double-sided adhesive layer such that one side of the adhesive layer may be fixed to bottom side 22, and the other adhesive side of the adhesive layer may have a temporary peel-off layer disposed thereon. Accordingly, the temporary peel-off layer may be removed from the adhesive layer when base 20 is ready to be affixed to the patient.

Top side 21 of base 20 may include a first mating surface, for example reclosable fasteners (e.g., Velcro^{®} made available by Velcro Industries N.V., United Kingdom), such that top side 21 may removably mate with the bottom surface of stabilizer 30 as described in more detail below. The entire surface of top side 21 may be covered with the first mating surface to provide ample space for stabilizer 30 to mate with base 20, such that stabilizer 30 may easily be repositioned until it is in a desired location with respect to opening 24 and the device insertion site.

In accordance with one aspect of the present invention, system 10 may be used in conjunction with a percutaneous suture-mediated closure device such as the Perclose ProGlide^{™} vascular closure device (made available by Abbott Laboratories, Chicago, Illinois). A percutaneous suture-mediated closure device delivers a single suture to close a puncture site, e.g., a catheter insertion site, in large vessels such as the femoral vein or artery, following catheterization procedures. Depending on the interventional procedure, more than one percutaneous suture-mediated closure device may be used. Accordingly, as the free ends of the one or more sutures are exposed external to the patient, the suture(s) may be rolled up and temporarily placed on the tips of fingers 23a, 23b. As shown in FIG. 2, a clear adhesive such as a Tegaderm^{™} (made available by 3M, Maplewood, Minnesota), e.g., strips 26a, 26b, may be temporarily fixed to the tips of fingers 23a, 23b, thereby securing the rolled up sutures therebetween. Once the procedure is complete, strips 26a, 26b may be removed to release the suture(s) so that the suture(s) may be used to close the puncture site.

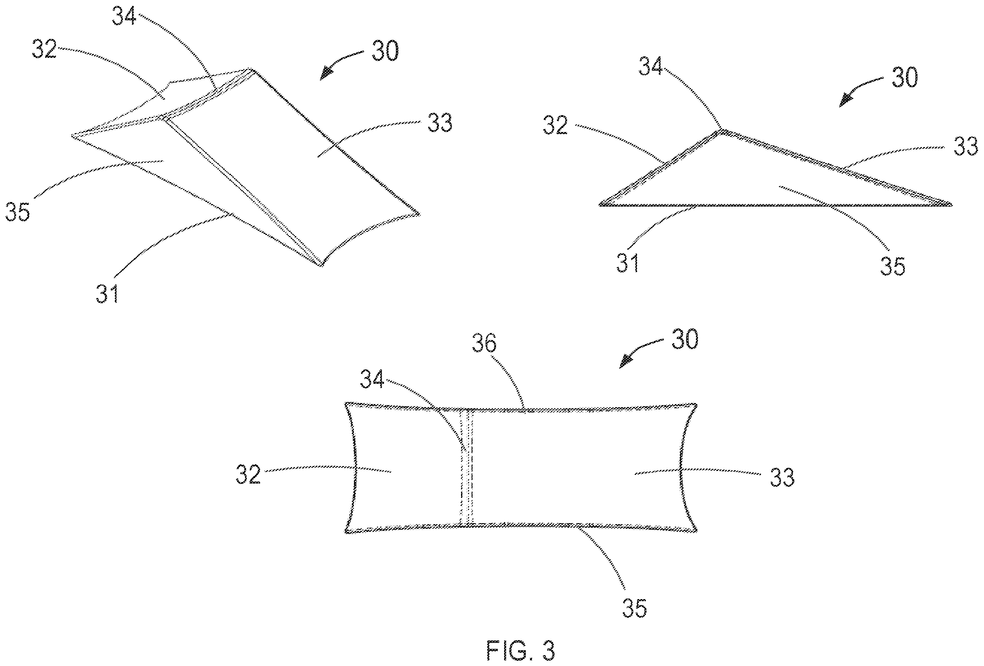

Referring now to FIG. 3, an exemplary stabilizer pad is provided. Stabilizer 30 may be formed of a conformable material, e.g., rubber, and may be sized to fit on base 20. For example, when base 20 is sized to be affixed to the patient's anterior quadriceps, stabilizer 30 may have a width of one to three inches, and a length of four to six inches. Stabilizer 30 has a geometry that permits a medical device entering the patient's body to extend from the insertion site at an angle corresponding to its insertion angle to thereby prevent stress at the insertion site, as well as unwanted bleeding. Stabilizer 30 also may have a geometry that permits a medical device to follow the stabilizer back toward the patient's body along a surface that prevents damage, e.g. kinking, of the device. For example, as shown in FIG. 3, stabilizer 30 may have bottom surface 31, first top surface 32, second top surface 33, lateral side surface 35, and lateral side surface 36.

Bottom surface 31 may be partially or entirely covered with a second mating surface, for example, reclosable fasteners (e.g., Velcro^{®} made available by Velcro Industries N.V., United Kingdom), such that bottom surface 31 may removably mate with the first mating surface of top side 21 of base 20. As will be understood by a person of ordinary skill in the art, the second mating surface may be made of a primary reclosable surface, e.g. loops, if the first mating surface is made of a secondary reclosable surface, e.g. hoops. As shown in FIG. 3, first top surface 32 may extend from a first end of bottom surface 31 at a predetermined angle, e.g., 20 to 40 degrees, preferably 30 degrees, such that the angle of first top surface 32 corresponds with the medical device's insertion angle, which may vary depending on the device used and the insertion site. First top surface 32 meets with second top surface 33 at apex 34, and second top surface 33 angles back toward to bottom surface at a predetermined angle relative to bottom surface 31, e.g., 10 to 30 degrees, preferably 20 degrees, selected to position the medical device back toward the patient's body without causing a severe change in angle which can damage the device. Accordingly, apex 34 may have a radius of curvature selected to prevent stress at the inflection point of the medical device as it extends from first top surface 32 to second top surface 33. In addition, apex 34 may be positioned at a height of, e.g., one inch from bottom surface 31. As will be understood by a person of ordinary skill in the art, the dimensions of stabilizer 30 described herein will depend on the medical device being used and the underlying procedure as well as the anatomical region of the patient system 10 is affixed thereto.

Moreover, as shown in FIG. 3, first top surface 32 and/or second top surface 33 may have a concave shape to facilitate positioning of the medical device. For example, the medical device may rest at the bottom of the concave geometry of first top surface 32 and/or second top surface 33. The degree of the concavity of first top surface 32 and/or second top surface 33 may be selected based on the size of the device used. In addition, lateral side surface 35 and/or lateral side surface 36 also may be covered within a mating surface such as reclosable fasteners, e.g., Velcro^{®}, for removably mating with one or more straps for securing the device to stabilizer 30 as described in further detail below. In some embodiments, stabilizer 30 may be affixed directly to the patient's skin without base 20. For example, bottom surface 31 of stabilizer 30 may include an adhesive that may be used to adhere stabilizer 30 directly to the patient's skin.

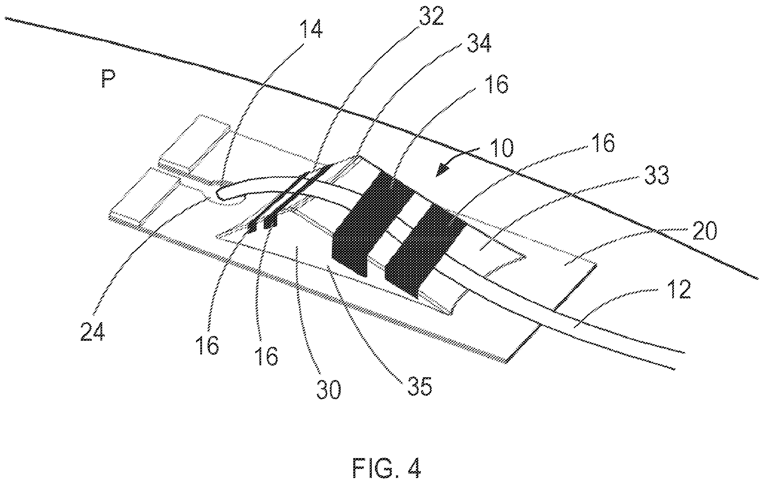

Referring now to FIG. 4, stabilization system 10 having base 20 and stabilizer 30 is illustrated in use for securing the extracorporeal portion of catheter 12 to patient P, e.g., the patient's anterior quadriceps. As shown in FIG. 4, base 20 is affixed to the patient via its adhesive bottom side, such that opening 24 is aligned with catheter insertion site 14 where catheter 12 enters patient P. In addition, stabilizer 30 is coupled to base 20 via the first and second mating surfaces described above, in a position such that first top surface 32 is adjacent opening 24. Accordingly, catheter 12 may extend from catheter insertion site 14 along first top surface 32 at an angle corresponding with its insertion angle, bend over apex 34 at a radius of curvature that prevents damage, e.g., kinking, of catheter 12, and continue extending along second top surface 33 of stabilizer 30.

Further, as shown in FIG. 4, stabilization system 10 may include one or more straps 16 for securing catheter 12 to stabilizer 30, and accordingly, patient P. One or more straps 16 may have a mating surface that will mate with the mating surface of lateral sides 35 and 36. For example, if lateral sides 35 and 36 are covered with a mating surface made of loops, then the bottom surface of one or more straps 16 will be made of hooks, and vice versa. Although FIG. 4 illustrates four straps 16, as will be understood by a person having ordinary skill in the art, more or less than four straps may be used. Each of one or more straps 16 are sized and shaped to secure catheter 12 to stabilization system 10. For example, each of one or more straps 16 may be four inches long and half an inch wide. As will be understood by a person of ordinary skill in the art, the dimensions of one or more straps 16 may vary depending on the catheter used, how many straps are needed, and the dimensions of stabilizer 30 selected.

In addition, one or more straps 16 may be removably coupled to stabilizer 30 over both first top surface 32 and second top surface 33 as illustrated in FIG. 4, or alternatively, one or more straps 16 may be removably coupled to stabilizer 30 over first top surface 32 or second top surface 33. In accordance with another aspect of the present invention, one or more strap 16 may be fixed to one lateral side surface, e.g., lateral side surface 36, and removably coupled to the other lateral side surface, e.g., lateral side surface 35, via the complementary loops and hooks mating surfaces described above.

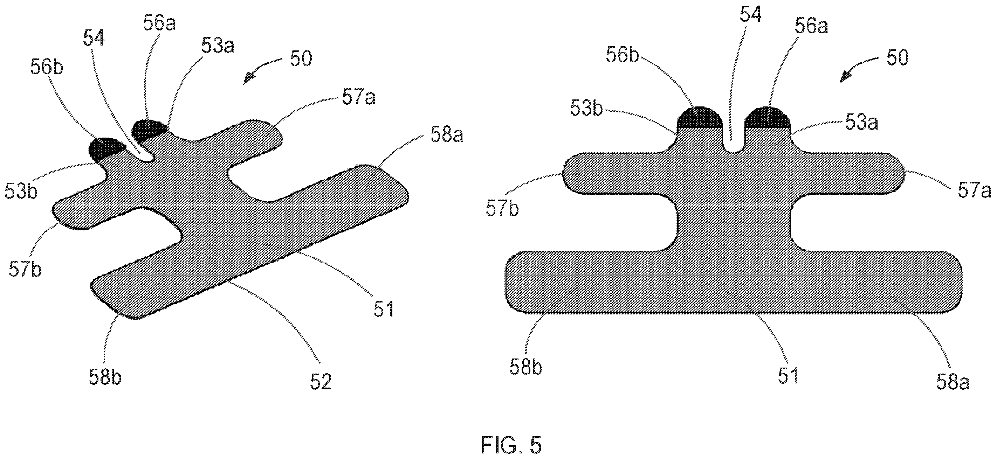

Referring now to FIG. 5, an alternative exemplary base is provided. Base 50 is constructed similar to base 20 of FIG. 2, except that base 50 has a different shape, e.g., having first pair of wings 57a, 57b and second pair of wings 58a, 58b. As shown in FIG. 5, first pair of wings 57a, 57b and second pair of wings 58a, 58b extend from the lateral edges of base 50 to facilitate affixing base 50 to the patient's body. Moreover, first pair of wings 57a, 57b, e.g., the wings closer to the device insertion site, may have a length that is less than the length of second pair of wings 58a, 58b, or vice versa, depending on the target area of the patient's body that base 50 will be affixed to.

Accordingly, base 50 may be sized and shaped to be affixed to various parts of the patient's body. For example, when sized to be affixed to the patient's anterior quadriceps, base 50 may have a length of seven to nine inches, a length spanning from wing 58a to wing 58b of fifteen to seventeen inches, a length spanning from wing 57a to wing 57b of eleven to thirteen inches, a width of second pair of wings 58a, 58b of two to three inches, and a width of first pair of wings 57a, 57b of one to two inches. As will be understood by a person of ordinary skill in the art, the dimensions of base 50 may be selected to comfortably adhere to the patient's body in the desired location.

As shown in FIG. 5, base 50 includes opening 54, defined by fingers 53a, 53b. Accordingly, opening 54 may receive the medical device before base 50 is affixed to the patient's body. Opening 54 may be sized and shaped to receive various sized devices depending on the underlying procedure. Like base 20, base 50 may include top side 51 having a first mating surface for mating with bottom surface 31 of stabilizer 30, and bottom side 52 that may be affixed to the patient's skin using methods known in the art, e.g., adhesion. In addition, when system 10 having base 50 and stabilizer 30 is used in conjunction with one or more percutaneous suture-mediated closure devices, the suture(s) may be rolled up and temporarily placed on the tips of fingers 53a, 53b, and a clear adhesive, e.g., strips 56a, 56b, may be temporarily fixed to the tips of fingers 53a, 53b, thereby securing the rolled up sutures therebetween. Once the procedure is complete, strips 56a, 56b may be removed to release the suture(s) so that the suture(s) may be knotted to close the puncture site.

While various illustrative embodiments of the invention are described above, it will be apparent to one skilled in the art that various changes and modifications may be made therein without departing from the invention. The appended claims are intended to cover all such changes and modifications that fall within the true scope of the invention.

## Claims

1. A stabilizer (30, 40) for securing a medical device to a patient, the stabilizer (30, 40) having a bottom surface (31), a first top surface (32) extending from a first end of the bottom surface (31) to an apex (34) of the stabilizer (30, 40), and a second top surface (33) extending from the apex (34) of the stabilizer (30, 40) to a second end of the bottom surface (31), the stabilizer (30, 40)configured to be affixed to a skin of the patient such that the first top surface (32) is adjacent to an insertion site of the patient,
wherein the medical device extends from the insertion site along the first top surface (32) over the apex (34) and along the second top surface (33) of the stabilizer (30, 40) toward the patient to thereby secure the medical device to the patient.

2. The stabilizer of claim 1, wherein the bottom surface (31) of the stabilizer (30, 40) comprises an adhesive configured to adhere the stabilizer (30, 40) directly to the skin of the patient.

3. The stabilizer of claim 1, wherein at least one of the first top surface (32) or the second top surface (33) comprises a concave geometry.

4. The stabilizer of claim 1, wherein the first top surface (32) extends from the first end of the bottom surface (31) at an angle corresponding with an insertion angle of the medical device into the patient, to thereby reduce bleeding.

5. The stabilizer of claim 1, wherein the first top surface (32) extends from the first end of the bottom surface (31) at an angle larger than an angle at which the second top surface (33) extends from the second end of the bottom surface (31),
and preferably
wherein the first top surface (32) extends from the first end of the bottom surface (31) at an angle of at least 30 degrees, and wherein the second top surface (33) extends from the second end of the bottom surface (31) at an angle of at least 20 degrees.

6. The stabilizer of claim 1, wherein the stabilizer (30, 40) comprises at least one of a closed cell foam, thermoplastic, rubber, or other elastomeric or plastic material.

7. The stabilizer of claim 1, wherein the apex (34) of the stabilizer (30, 40) comprises a radius of curvature configured to prevent kinking of the medical device,
and preferably
wherein the apex (34) of the stabilizer (30, 40) is at least one inch from the bottom surface (31) of the stabilizer (30, 40).

8. A stabilization system (10) comprising the stabilizer (30, 40) of claim 1, and one or more straps (16) configured to be removably coupled to the stabilizer (30, 40) such that the medical device is positioned between the stabilizer (30, 40) and the one or more straps (16),
and preferably
wherein the one or more straps (16) are configured to be removably coupled to the stabilizer (30, 40) over at least one of the first top surface (32) or the second top surface (33),
and preferably
wherein the one or more straps (16) comprise a primary mating surface, and wherein lateral sides (35, 36) of the stabilizer (30, 40) comprise a secondary mating surface configured to mate with the primary mating surface,
and preferably
wherein an end of the one or more straps (16) is fixed to a first lateral side (35) of the stabilizer (30, 40), and an opposite end of the one or more straps (16) is removably coupled to a second lateral side (36) of the stabilizer (30, 40).

9. A stabilization system (10) comprising the stabilizer (30, 40) of claim 1 and a base (20, 50) having a bottom side (22, 52) and a top side (21, 51), the bottom side configured to be affixed to the skin of the patient, the top side comprising a first mating surface, the base (20, 50) comprising an opening (24, 54) sized and shaped to receive the medical device therethrough,
wherein the bottom surface (31) of the stabilizer (30, 40) comprises a second mating surface configured to mate with the first mating surface of the base (20, 50) such that the stabilizer (30, 40) is affixed to the skin of the patient via the base (20, 50) and the first top surface (32) of the stabilizer (30, 40) is adjacent to the opening,
and preferably
wherein the bottom side of the base (20, 50) comprises an adhesive configured to adhere the base (20, 50) to the skin of the patient,
and preferably
wherein the base (20, 50) is sized and shaped to be affixed to a vascular access site of the patient selected from a group consisting of a femoral, popliteal, brachial, axial, tibial, or carotid.

10. The system of claim 9, wherein the base comprises two fingers (23a, 23b, 53a, 53b) defining the opening,
and preferably
wherein the two fingers are configured to be coupled to one or more sutures via an external sterile adhesive.

11. The system of claim 9, wherein the base (20, 50) comprises a plurality of wings (57a, 57b, 58a, 58b) extending from a lateral side of the base (20, 50),
and preferably
wherein a first wing (57a, 57b) of the plurality of wings has a length smaller than a length of a second wing (58a, 58b) of the plurality of wings.

12. The system of claim 9, wherein the stabilizer (30, 40) is configured to be decoupled from the base (20, 50), reoriented 180 degrees, and recoupled to the base (20, 50).

13. A stabilization system (10) comprising the stabilizer (30, 40) of claim 1, wherein the system is configured to secure a medical device selected from a group consisting of a ventricular support catheter, an intra-aortic balloon pump, an extracorporeal membrane oxygenation, ECMO, machine, a device for catheter-assisted thrombolysis, a temperature control therapy device, a cardiopulmonary support catheter, a catheter-directed thrombolysis system, a pulmonary artery catheter, or a temporary pacing catheter.

14. A method for securing a medical device to a patient, the method comprising:
selecting a stabilization system (10) comprising a stabilizer (30, 40), the stabilizer (30, 40) comprising a first top surface (32) extending from a bottom surface (31) of the stabilizer (30, 40) to an apex (34) and a second top surface (33) extending from the apex (34) to the bottom surface (31) of the stabilizer (30, 40); and
affixing the stabilization system to a skin of the patient such that the medical device extends from an insertion site of the patient along the first top surface (32), over the apex (34), and along the second top surface (33) of the stabilizer (30, 40) toward the patient to thereby secure the medical device to the patient.

15. The method of claim 14, wherein the stabilization system further comprises a base (20, 50), the base (20, 50) comprising an opening (24, 54) configured to receive the medical device therethrough, and wherein affixing the stabilization system to the skin of the patient comprises affixing the base (20, 50) to the skin of the patient and coupling the stabilizer (30, 40) to the base (20, 50) such that the top surface of the stabilizer (30, 40) is adjacent to the opening of the base (20, 50).

## Patentansprüche

1. Stabilisator (30, 40) zum Sichern einer medizinischen Vorrichtung an einem Patienten, wobei der Stabilisator (30, 40) eine untere Fläche (31), eine erste obere Fläche (32), die sich von einem ersten Ende der unteren Fläche (31) zu einem Scheitel (34) des Stabilisators (30, 40) erstreckt und eine zweite obere Fläche (33) aufweist, die sich von dem Scheitel (34) des Stabilisators (30, 40) zu einem zweiten Ende der unteren Fläche (31) erstreckt, wobei der Stabilisator (30, 40) konfiguriert ist, an einer Haut des Patienten befestigt zu werden, so dass die erste obere Fläche (32) zu einer Einführungsstelle des Patienten benachbart ist,
wobei sich die medizinische Vorrichtung von der Einführungsstelle entlang der ersten oberen Fläche (32) über den Scheitel (34) und entlang der zweiten oberen Fläche (33) des Stabilisators (30, 40) zum Patienten erstreckt, um dadurch die medizinische Vorrichtung am Patienten zu sichern.

2. Stabilisator nach Anspruch 1, wobei die untere Fläche (31) des Stabilisators (30, 40) einen Klebstoff aufweist, der konfiguriert ist, den Stabilisator (30, 40) direkt auf die Haut des Patienten zu kleben.

3. Stabilisator nach Anspruch 1, wobei mindestens eine der ersten oberen Fläche (32) oder der zweiten oberen Fläche (33) eine konkave Geometrie aufweist.

4. Stabilisator nach Anspruch 1, wobei sich die erste obere Fläche (32) vom ersten Ende der unteren Fläche (31) in einem Winkel erstreckt, der einem Einführungswinkel der medizinischen Vorrichtung in den Patienten entspricht, um dadurch Blutungen zu verringern.

5. Stabilisator nach Anspruch 1, wobei sich die erste obere Fläche (32) von dem ersten Ende der unteren Fläche (31) in einem Winkel erstreckt, der größer ist als der Winkel, in dem sich die zweite obere Fläche (33) von dem zweiten Ende der unteren Fläche (31) erstreckt, und vorzugsweise
wobei sich die erste obere Fläche (32) von dem ersten Ende der unteren Fläche (31) in einem Winkel von mindestens 30 Grad erstreckt, und wobei sich die zweite obere Fläche (33) von dem zweiten Ende der unteren Fläche (31) in einem Winkel von mindestens 20 Grad erstreckt.

6. Stabilisator nach Anspruch 1, wobei der Stabilisator (30, 40) mindestens einen geschlossenzelligen Schaumstoff, Thermoplast, Gummi oder ein anderes elastomeres oder plastisches Material aufweist.

7. Stabilisator nach Anspruch 1, wobei der Scheitel (34) des Stabilisators (30, 40) einen Krümmungsradius aufweist, der konfiguriert ist, ein Abknicken der medizinischen Vorrichtung zu verhindern,
und vorzugsweise
wobei der Scheitel (34) des Stabilisators (30, 40) mindestens einen Zoll von der unteren Fläche (31) des Stabilisators (30, 40) entfernt ist.

8. Stabilisierungssystem (10), das den Stabilisator (30, 40) nach Anspruch 1 und einen oder mehrere Riemen (16) aufweist, die konfiguriert sind, abnehmbar mit dem Stabilisator (30, 40) gekoppelt zu werden, so dass die medizinische Vorrichtung zwischen dem Stabilisator (30, 40) und dem einen oder den mehreren Riemen (16) positioniert ist,
und vorzugsweise
wobei der eine oder die mehreren Riemen (16) konfiguriert sind, mit dem Stabilisator (30, 40) über mindestens eine der ersten oberen Fläche (32) oder der zweiten oberen Fläche (33) lösbar gekoppelt zu werden,
und vorzugsweise
wobei der eine oder die mehreren Riemen (16) eine primäre Passfläche aufweisen, und wobei die lateralen Seiten (35, 36) des Stabilisators (30, 40) eine sekundäre Passfläche aufweisen, die so konfiguriert ist, dass sie mit der primären Passfläche zusammenpasst, und vorzugsweise
wobei ein Ende des einen oder der mehreren Riemen (16) an einer ersten lateralen Seite (35) des Stabilisators (30, 40) befestigt ist und ein gegenüberliegendes Ende des einen oder der mehreren Riemen (16) lösbar mit einer zweiten lateralen Seite (36) des Stabilisators (30, 40) gekoppelt ist.

9. Stabilisierungssystem (10), das den Stabilisator (30, 40) nach Anspruch 1 und eine Basis (20, 50) mit einer Unterseite (22, 52) und einer Oberseite (21, 51) aufweist, wobei die Unterseite konfiguriert ist, an der Haut des Patienten befestigt zu werden, wobei die Oberseite eine erste Passfläche aufweist, wobei die Basis (20, 50) eine Öffnung (24, 54) aufweist, die bemessen und geformt ist, die medizinische Vorrichtung durch sie hindurch aufzunehmen,
wobei die untere Fläche (31) des Stabilisators (30, 40) eine zweite Passfläche aufweist, die so konfiguriert ist, dass sie mit der ersten Passfläche der Basis (20, 50) zusammenpasst, so dass der Stabilisator (30, 40) über die Basis (20, 50) an der Haut des Patienten befestigt ist und die erste obere Fläche (32) des Stabilisators (30, 40) zur Öffnung benachbart ist,
und vorzugsweise
wobei die Unterseite der Basis (20, 50) einen Klebstoff aufweist, der konfiguriert ist, die Basis (20, 50) an die Haut des Patienten zu kleben,
und vorzugsweise
wobei die Basis (20, 50) bemessen und geformt ist, an einer vaskulären Zugangsstelle des Patienten befestigt zu werden, die aus einer Gruppe ausgewählt ist, die aus einer femoral, popliteal, brachial, axial, tibial oder karotid besteht.

10. System nach Anspruch 9, wobei die Basis zwei Finger (23a, 23b, 53a, 53b) aufweist, die die Öffnung definieren,
und vorzugsweise
wobei die beiden Finger konfiguriert sind, über einen externen sterilen Klebstoff mit einer oder mehreren Nähten gekoppelt zu werden.

11. System nach Anspruch 9, wobei die Basis (20, 50) mehrere Flügel (57a, 57b, 58a, 58b) aufweist, die sich von einer lateralen Seite der Basis (20, 50) erstrecken,
und vorzugsweise
wobei ein erster Flügel (57a, 57b) der mehreren Flügel eine Länge aufweist, die kleiner ist als eine Länge eines zweiten Flügels (58a, 58b) der mehreren Flügel.

12. System nach Anspruch 9, wobei der Stabilisator (30, 40) konfiguriert ist, von der Basis (20, 50) entkoppelt, um 180 Grad umorientiert und wieder mit der Basis (20, 50) gekoppelt zu werden.

13. Stabilisierungssystem (10), das den Stabilisator (30, 40) von Anspruch 1 aufweist, wobei das System konfiguriert ist, eine medizinische Vorrichtung zu sichern, die aus einer Gruppe ausgewählt ist, die aus einem ventrikulären Unterstützungskatheter, einer intraaortalen Ballonpumpe, einer extrakorporalen Membranoxygenierung, ECMO, Maschine, einer Vorrichtung zur kathetergestützten Thrombolyse, einer Temperaturregelungs-Therapievorrichtung, einem kardiopulmonalen Unterstützungskatheter, einem kathetergesteuerten Thrombolysesystem, einem Pulmonalarterienkatheter oder einem temporären Schrittmacherkatheter besteht.

14. Verfahren zum Sichern einer medizinischen Vorrichtung an einem Patienten, wobei das Verfahren aufweist:
Auswählen eines Stabilisierungssystems (10), das einen Stabilisator (30, 40) aufweist, wobei der Stabilisator (30, 40) eine erste obere Fläche (32), die sich von einer unteren Fläche (31) des Stabilisators (30, 40) zu einem Scheitel (34) erstreckt, und eine zweite obere Fläche (33) aufweist, die sich von dem Scheitel (34) zu der unteren Fläche (31) des Stabilisators (30, 40) erstreckt; und
Befestigen des Stabilisierungssystems an einer Haut des Patienten, so dass sich die medizinische Vorrichtung von einer Einführungsstelle des Patienten entlang der ersten oberen Fläche (32), über den Scheitel (34) und entlang der zweiten oberen Fläche (33) des Stabilisators (30, 40) zu dem Patienten erstreckt, um dadurch die medizinische Vorrichtung an dem Patienten zu sichern.

15. Verfahren nach Anspruch 14, wobei das Stabilisierungssystem ferner eine Basis (20, 50) aufweist, wobei die Basis (20, 50) eine Öffnung (24, 54) aufweist, die konfiguriert ist, die medizinische Vorrichtung durch sie hindurch aufzunehmen, und wobei das Befestigen des Stabilisierungssystems an der Haut des Patienten das Befestigen der Basis (20, 50) an der Haut des Patienten und das Koppeln des Stabilisators (30, 40) mit der Basis (20, 50) aufweist, so dass die obere Fläche des Stabilisators (30, 40) zur Öffnung der Basis (20, 50) benachbart ist.

## Revendications

1. Stabilisateur (30, 40) pour fixer un dispositif médical à un patient, ledit stabilisateur (30, 40) ayant une surface inférieure (31), une première surface supérieure (32) s'étendant d'une première extrémité de la surface inférieure (31) à un sommet (34) du stabilisateur (30, 40), et une deuxième surface supérieure (33) s'étendant du sommet (34) du stabilisateur (30, 40) à une deuxième extrémité de la surface inférieure (31), ledit stabilisateur (30, 40) étant prévu pour être fixé sur la peau du patient de sorte que la première surface supérieure (32) est adjacente à un site d'insertion du patient,
où le dispositif médical s'étend à partir du site d'insertion, le long de la première surface supérieure (32) au-delà du sommet (34) et le long de la deuxième surface supérieure (33) du stabilisateur (30, 40) vers le patient afin de fixer le dispositif médical au patient.

2. Stabilisateur selon la revendication 1, où la surface inférieure (31) du stabilisateur (30, 40) comprend un adhésif prévu pour une adhérence directe du stabilisateur (30, 40) sur la peau du patient.

3. Stabilisateur selon la revendication 1, la première surface supérieure (32) et/ou la deuxième surface supérieure (33) présentent une géométrie concave.

4. Stabilisateur selon la revendication 1, où la première surface supérieure (32) s'étend à partir de la première extrémité de la surface inférieure (31) suivant un angle correspondant à l'angle d'insertion du dispositif médical dans le patient pour limiter les saignements.

5. Stabilisateur selon la revendication 1, où la première surface supérieure (32) s'étend à partir de la première extrémité de la surface inférieure (31) suivant un angle supérieur à l'angle suivant lequel la deuxième surface supérieure (33) s'étend à partir de la deuxième extrémité de la surface inférieure (31),
et où, de préférence,
la première surface supérieure (32) s'étend à partir de la première extrémité de la surface inférieure (31) suivant un angle égal ou supérieur à 30 degrés, et où la deuxième surface supérieure (33) s'étend à partir de la deuxième extrémité de la surface inférieure (31) suivant un angle égal ou supérieur à 20 degrés.

6. Stabilisateur selon la revendication 1, où le stabilisateur (30, 40) comprend au moins un matériau entre une mousse à cellules fermées, un thermoplastique, un caoutchouc ou un autre matériau élastomère ou plastique.

7. Stabilisateur selon la revendication 1, où le sommet (34) du stabilisateur (30, 40) présente un rayon de courbure prévu pour empêcher la torsion du dispositif médical,
et où, de préférence,
le sommet (34) du stabilisateur (30, 40) est distant d'au moins un pouce de la surface inférieure (31) du stabilisateur (30, 40).

8. Système de stabilisation (10), comprenant le stabilisateur (30, 40) selon la revendication 1, et une ou plusieurs sangles (16) prévues pour être raccordées de manière amovible au stabilisateur (30, 40), de sorte que le dispositif médical est positionné entre le stabilisateur (30, 40) et la ou les sangles (16),
et où, de préférence,
la ou les sangles (16) sont prévues pour être raccordées de manière amovible au stabilisateur (30, 40) sur la première surface supérieure (32) et/ou la deuxième surface supérieure (33),
et où, de préférence,
la ou les sangles (16) présentent une surface de contact primaire, et où les faces latérales (35, 36) du stabilisateur (30, 40) présentent une surface de contact secondaire prévue pour être mise en contact avec la surface de contact primaire,
et où, de préférence,
une extrémité de la ou des sangles (16) est fixée à une première surface latérale (35) du stabilisateur (30, 40), et une extrémité opposée de la ou des sangles (16) est mise en contact de manière amovible avec une deuxième surface latérale (36) du stabilisateur (30, 40).

9. Système de stabilisation (10) comprenant le stabilisateur (30, 40) selon la revendication 1 et une base (20, 50) ayant une face inférieure (22, 52) et une face supérieure (21, 51), la face inférieure étant prévue pour être fixée sur la peau du patient, la face supérieure présentant une première surface de contact, la base (20, 50) présentant une ouverture (24, 54) dimensionnée et formée pour recevoir le dispositif médical,
où la surface inférieure (31) du stabilisateur (30, 40) comprend une deuxième surface de contact prévue pour être mise en contact avec la première surface de contact de la base (20, 50), de sorte que le stabilisateur (30, 40) est fixé sur la peau du patient par la base (20, 50) et que la première surface supérieure (32) du stabilisateur (30, 40) est adjacente à l'ouverture,
et où, de préférence,
la face inférieure de la base (20, 50) comprend un adhésif prévu faire adhérer la base (20, 50) sur la peau du patient,
et où, de préférence,
la base (20, 50) est dimensionnée et formée pour être fixée sur un site d'accès vasculaire du patient sélectionné dans un groupe constitué d'un accès fémoral, poplité, brachial, axial, tibial ou carotidien.

10. Système selon la revendication 9, où la base comprend deux doigts (23a, 23b, 53a, 53b) définissant l'ouverture,
et où, de préférence,
les deux doigts sont prévus pour être raccordés à une ou plusieurs sutures au moyen d'un adhésif stérile externe.

11. Système selon la revendication 9, où la base (20, 50) comprend une pluralité de branches (57a, 57b, 58a, 58b) s'étendant depuis un côté latéral de la base (20, 50),
et où, de préférence,
une première branche (57a, 57b) de la pluralité de branches a une longueur inférieure à la longueur d'une deuxième branche (58a, 58b) de la pluralité de branches.

12. Système selon la revendication 9, où le stabilisateur (30, 40) est prévu pour être détaché de la base (20, 50), réorienté de 180 degrés, et rattaché à la base (20, 50).

13. Système de stabilisation (10), comprenant le stabilisateur (30, 40) selon la revendication 1, où ledit système est prévu pour fixer un dispositif médical sélectionné dans un groupe constitué d'un cathéter d'assistance ventriculaire, d'une pompe à ballon intra-aortique, d'une machine d'oxygénation par membrane extracorporelle, ECMO, d'un dispositif de thrombolyse assistée par cathéter, d'un dispositif de thermothérapie, d'un cathéter d'assistance cardio-pulmonaire, d'un système de thrombolyse dirigée par cathéter, d'un cathéter d'artère pulmonaire ou d'un cathéter stimulateur temporaire.

14. Procédé de fixation d'un dispositif médical sur un patient, ledit procédé comprenant :
la sélection d'un système de stabilisation (10) comprenant un stabilisateur (30, 40), ledit stabilisateur (30, 40) ayant une première surface supérieure (32) s'étendant d'une surface inférieure (31) du stabilisateur (30, 40) vers un sommet (34), et une deuxième surface supérieure (33) s'étendant du sommet (34) à la surface inférieure (31) du stabilisateur (30, 40) ; et
la fixation du système de stabilisation sur la peau du patient de sorte que le dispositif médical s'étend à partir d'un site d'insertion du patient le long de la première surface supérieure (32) au-delà du sommet (34) et le long de la deuxième surface supérieure (33) du stabilisateur (30, 40) vers le patient afin de fixer le dispositif médical au patient.

15. Procédé selon la revendication 14, où le système de stabilisation comprend en outre une base (20, 50), ladite base (20, 50) présentant une ouverture (24, 54) prévue pour recevoir le dispositif médical, et où la fixation du système de stabilisation sur la peau du patient comprend la fixation de la base (20, 50) sur la peau du patient et le raccordement du stabilisateur (30, 40) à la base (20, 50) de sorte que la surface supérieure du stabilisateur (30, 40) est adjacente à l'ouverture de la base (20, 50).
